# FASCICULE DE BREVET EUROPEEN

(11) **EP 0 765 850 B1**
(45) Date de publication et mention de la délivrance du brevet: **03.01.2001**
(21) Numéro de dépôt: 96402017.6
(22) Date de dépôt: 24.09.1996
(51) Int. Cl.: C07C 15/08

(54) **Production de paraxylène à partir d'un effluent de dismutation parasélective du toluène par un procédé de cristallisation associé à une adsorption en lit mobile simulé**
Verfahren zur Herstellung von para-Xylol aus dem Produkt einer paraselektiven Disproportionierung von Toluol durch ein Kristallisierungsverfahren in Zusammenhang mit einer Adsorptionstufe in einem simulierten Bewegungsbett
Process for the preparation of para-xylene from an effluent of the paraselective dismutation of toluene by a cristallisation process linked to adsorption in a simulated moving bed

(30) Priorité: 29.09.1995 FR 9511489
(43) Date de publication de la demande: 02.04.1997
(73) Titulaire: INSTITUT FRANCAIS DU PETROLE, 92502 Rueil-Malmaison (FR)
(72) Inventeur: Mikitenko, Paul, 78590 Noisy le Roy (FR); Hotier, Gérard, 92500 Rueil Malmaison (FR)

(56) Documents cités:
- EP-A- 0 531 191
- US-A- 5 329 060

## Description

La présente invention concerne un procédé de production et de séparation amélioré des hydrocarbures aromatiques en C8 à partir d'une charge riche en paraxylène, par exemple telle qu'une coupe C8 du procédé Mobil de dismutation du toluène.

Il est reconnu depuis longtemps que certains matériaux zéolithiques catalysent certaines conversions d'hydrocarbures comme il est décrit par exemple dans le brevet US n° 4 305 808. Les conversions d'hydrocarbures revendiquées sont particulièrement l'alkylation, la transalkylation et la dismutation. La dismutation du toluène est revendiquée, par exemple, dans les brevets US n° 4 052 476 ; 4 007 231 ; 4 011 276 ; 4 016 219 et 4 029 716.

Dans un article publié dans Oil and Gas Journal vol. 69 n°48 (1971), Grandio et coll. décrit un procédé de dismutation du toluène en phase liquide utilisant des catalyseurs zéolithiques en absence d'hydrogène.

Il faut noter que dans les premiers procédés connus, la composition des xylènes obtenus est telle qu'elle correspond à l'équilibre thermodynamique à la température opératoire, soit de 20 à 25 % de p-xylène sur la totalité des xylènes et de l'éthylbenzène.

Des brevets plus récents, par exemple US 4 380 685 décrivent l'alkylation, la transalkylation et la dismutation parasélective de composés aromatiques substitués pour former des dialkylbenzènes. Dans le cas de la dismutation du toluène la teneur en p-xylène de l'effluent produit excède l'équilibre thermodynamique. Le catalyseur utilisé est à base de zéolithes caractérisées par un indice de contrainte de 1 à 12, un rapport silice/alumine d'au moins 12/1 et comprenant divers métaux et du phosphore ; les zéolithes utilisées sont par exemple des ZSM5, ZSM11, ZSM12 ou ZSM35. De plus, ces catalyseurs doivent être traités pour rendre la diffusion plus restreinte à travers les pores du cristal de l'ortho- et du métaxylène et réduire la réisomérisation du p-xylène formé. Ce traitement consiste essentiellement en un précokage effectué dans des conditions bien déterminées éventuellement accompagné de l'addition d'une petite quantité d'oxyde difficilement réductible (antimoine, phosphore, bore ou magnésium) ou d'un traitement de surface. Ces méthodes de préparation d'un catalyseur efficace sont décrites par exemple dans le brevet US 5173 461.

Le procédé employant ces catalyseurs, appelé MSTDP est décrit dans deux publications :
Selective toluene disproportionation process proven at Italian refinery,
   Gorra F., Breckenridge L.L., Guy W.M., Sailor R.A.,
   Oil and Gas Journal V90, N41, 60-67 (1992) et
Mobil's toluene to PX process proves itself
   Mobil Research and Development Corp.
   European Chemical News V54, N1418 (1990).

Ces publications décrivent une production de xylènes contenant 80-95 % de p-xylène pour une conversion de 30 % de toluène par passe.

De nombreux brevets revendiquent ce procédé et décrivent des préparations de catalyseur, par exemple EP 26 962 ; US 4 260 843 ; US 4 274 982 ; US 4 908 342 ; US 5 173 461 ; WO 93/17.987.

Dans tous ces cas l'effluent de dismutation a une composition simplifiée voisine de : toluène 70 % - benzène 15 % - xylènes 15 % - la composition de la fraction xylènes est p-xylène 85 %, O + m-xylène et éthylbenzène 15 %.

La présente invention est relative à un procédé amélioré pour la séparation du mélange C8 aromatiques pour obtenir du p-xylène de pureté suffisante pour la production, par exemple, d'acide téréphtalique. Une méthode connue de séparation du p-xylène à partir de la coupe C8 aromatiques consiste à effectuer une cristallisation fractionnée ; parmi les procédés existants, on peut citer ceux de Chevron, Krupp, Amoco, Maruzen et Arco (U.S.P. n° 3 177 255 et 3 467 724. Ces procédés utilisent la procédure suivante : la charge contenant au moins 20 % de p-xylène est refroidie entre -50 et -70°C ce qui provoque une cristallisation, les cristaux contiennent de 85 à 90 % de p-xylène et les liqueurs mères de 7 à 8 % ; les cristaux sont refondus et recristallisés à -10°C. Après filtration des cristaux et lavage, par exemple, au toluène, on obtient du paraxylène à une pureté de 99,5 %.

Une autre méthode de séparation du p-xylène d'un mélange d'aromatique en C8 est la méthode chromatographique en phase liquide dite à contre-courant simulé revendiquée dans le brevet USP n° 2 985 589 qui utilisent des zéolithes pour absorber sélectivement le p-xylène. Les procédés Parex et Aromax utilisent cette méthode ou celle du co-courant simulé (USP n° 4 402 832).

Les avantages et inconvénients de ces procédés ont été largement étudiés et décrits dans différents cas.

L'arrière plan technologique est illustré par ailleurs par les brevets US-A-5329 060 et EP-A-531191, qui décrivent une combinaison d'étapes d'adsorption, de cristallisation et d'isomérisation de coupes aromatiques en C8 pour obtenir du paraxylène de très haute pureté.

La particularité du mélange obtenu, en sortie d'un réacteur de dismutation parasélective dans le MSTDP, après séparation des hydrocarbures plus légers par distillation, est d'être particulièrement riche en p-xylène puisqu'il contient 75 à 85 % de p-xylène.

Ce mélange peut être traité de différentes façons :
1) Par cristallisation à haute température
   On entend par haute température, une température comprise par exemple entre +10°C et -25°C. Le rendement en p-xylène pur est voisin de 83 % lorsque la teneur en paraxylène de la coupe C8 de l'effluent est de 80 % poids. Ce rendement est limité par le fait que la liqueur mère contient encore, du fait de l'équilibre thermodynamique liquide-solide, du paraxylène à une teneur supérieure à 40 %.
2) Par cristallisation à deux températures
   Dans ce cas une première cristallisation à une température comprise entre +10 et -25°C conduit à la production de paraxylène pur, la liqueur mère est recristallisée à une température plus basse (-50 à -70°C), les cristaux riches en p-xylène sont recyclés à la charge de la première cristallisation, la liqueur-mère de la seconde cristallisation ne contient plus qu'environ 10 % poids de p-xylène.

Le rendement global en paraxylène de la cristallisation en deux étapes pour une charge initiale de 80 % poids de paraxylène est de 97 % poids environ.

L'inconvénient de ce procédé est la forte consommation en énergie.

L'objet de l'invention est de remédier aux inconvénients mentionnés ci-dessus.

Plus précisément, l'invention concerne un procédé de préparation de paraxylène à partir d'une charge de toluène comprenant :
a) une étape de dismutation parasélective du toluène en présence d'hydrogène et d'un catalyseur dans une zone de dismutation délivrant un effluent contenant du benzène, du toluène, des xylènes enrichis en paraxylène, de l'éthylbenzène et des hydrocarbures aromatiques ayant au moins 9 atomes de carbone ;
b) une étape de distillation de l'effluent dans au moins deux colonnes de distillation délivrant séparément du benzène, du toluène et des xylènes ;
c) une étape de cristallisation des xylènes à haute température, de préférence comprise entre +10 et -30°C, dans au moins une zone de cristallisation délivrant du paraxylène de très haute pureté et une liqueur mère appauvrie en paraxylène, le procédé étant caractérisé en ce que :
d) on met en contact dans au moins une zone d'adsorption en lit mobile simulé ladite liqueur mère avec un lit d'adsorbant zéolithique en présence d'un désorbant qui est le toluène, dans des conditions telles qu'on obtient une première fraction appauvrie en paraxylène et contenant du toluène et une deuxième fraction enrichie en paraxylène contenant du toluène ;
e) on recycle au moins une partie de ladite deuxième fraction dans la zone de cristallisation après en avoir distillé sensiblement tout le toluène.

Par cristallisation à haute température, on entend une cristallisation dans au moins un cristalliseur pour chaque étape de cristallisation, d'une solution ou suspension de paraxylène, déjà enrichie en paraxylène, qui correspond à ce que la littérature appelle une étape de purification. Par exemple, le brevet US 2 866 833 incorporé comme référence, mentionne une étape de purification du paraxylène à haute température pouvant aller jusqu'à une température de -34°C.

Selon une caractéristique de l'invention, l'étape (b) de distillation peut comprendre la distillation de l'effluent de l'étape (a) dans une première colonne de distillation (C1) qui délivre un distillat contenant du benzène et un premier résidu, la distillation du premier résidu dans une deuxième colonne de distillation (C2) qui délivre un distillat contenant du toluène et un second résidu, et la distillation du second résidu dans une troisième colonne de distillation (C3) qui délivre un distillat contenant les xylènes et un troisième résidu contenant les hydrocarbures aromatiques à au moins 9 atomes de carbone et dans lequel une partie au moins de ladite deuxième fraction est distillée dans la deuxième colonne de distillation (C2).

Le troisième résidu peut être distillé en vue d'obtenir un distillat contenant de l'orthoxylène et un résidu contenant les hydrocarbures à au moins 9 atomes de carbone.

Selon une variante, l'étape (b) de distillation peut comprendre la distillation de l'effluent de l'étape (a), dans une première colonne de distillation (C1) qui délivre d'une part, un distillat (BT) contenant du benzène et du toluène et d'autre part un résidu contenant les xylènes et les hydrocarbures à au moins 9 atomes de carbone, on soumet ledit résidu à l'étape (c) de cristallisation, on distille une partie au moins de ladite deuxième fraction enrichie en paraxylène dans la colonne de distillation (C1), on distille le distillat (BT) contenant le benzène et le toluène dans une colonne de distillation (C2) de façon à récupérer en tant que distillat du benzène et comme résidu du toluène et dans lequel une partie au moins du distillat (BT) de la colonne (C1) est utilisée pour apporter la chaleur nécessaire au rebouilleur de la colonne (C2).

On peut envoyer le résidu de la colonne (C1) contenant les xylènes, l'éthylbenzène et les hydrocarbures à au moins 9 atomes de carbone directement dans au moins une zone de cristallisation, mais on peut aussi distiller ce résidu dans une colonne (C3) et récupérer un distillat contenant les xylènes et l'éthylbenzène que l'on soumet à l'étape (c) de cristallisation. Les hydrocarbures à au moins 9 atomes de carbone sont éliminés en tant que résidu.

Selon une autre caractéristique secondaire, une partie au moins du toluène provenant de la colonne de distillation C2 peut être recyclée à la zone d'adsorption, la partie restante étant recyclée dans la zone de dismutation parasélective.

Selon une première caractéristique relative à l'adsorption, la première fraction appauvrie en paraxylène contenant le toluène et les autres hydrocarbures aromatiques à 8 atomes de carbone et plus peut être distillée dans une colonne de distillation (C4) qui délivre, comme distillat, du toluène que l'on recycle au moins en partie dans la zone d'adsorption et un résidu sensiblement dépourvu de toluène contenant la première fraction c'est-à-dire l'orthoxylène, le métaxylène et l'éthylbenzène et éventuellement les hydrocarbures à au moins 9 atomes de carbone.

Lorsque les hydrocarbures à au moins 9 atomes de carbone n'ont pas été éliminés en amont de la zone de cristallisation, on peut les soutirer dans une colonne de distillation (C3) en aval de la colonne (C4), en tant que résidu.

Selon une deuxième caractéristique particulièrement avantageuse relative à l'adsorption une partie au moins de la deuxième fraction enrichie en paraxylène et contenant du toluène peut être recyclée dans la colonne (C1) de distillation lorsque le benzène seul est récupéré comme distillat de la colonne (C2). Une autre variante peut consister à recycler ladite deuxième fraction dans la colonne (C2) lorsque le benzène seul est récupéré comme distillat dans la colonne (C1).

L'étape de cristallisation des xylènes récupérés après distillation avec ou sans hydrocarbures à au moins 9 atomes de carbone peut être réalisée selon plusieurs variantes :
- Selon une première variante, on peut cristalliser les xylènes selon l'étape (c), dans une zone de cristallisation qui délivre une suspension de cristaux de paraxylène dans la liqueur mère, on sépare lesdits cristaux de la liqueur mère dans une zone de séparation, on lave les cristaux avec un solvant de lavage adéquat, on récupère la liqueur mère que l'on envoie dans la zone d'adsorption selon l'étape (d) et les cristaux de paraxylène de très haute pureté et on récupère une liqueur de lavage que l'on recycle dans la zone de distillation si le solvant de lavage contient du toluène ou dans la zone de cristallisation ou dans la zone d'adsorption si le solvant de lavage est du paraxylène fondu.
- Selon une deuxième variante qui peut être préférée avec certains types de cristalliseurs, par exemple les cristalliseurs raclés, on peut cristalliser les xylènes selon l'étape (c) dans au moins deux zones de cristallisation dont l'une est à une température plus froide que l'autre, on récupère la liqueur mère provenant de la zone de cristallisation la plus froide et on l'envoie selon l'étape (d) dans la zone d'adsorption.

Quelle que soit la variante, le paraxylène est cristallisé comme il a été indiqué ci-avant, à haute température, par exemple entre +10 et -34°C et en particulier entre +10 et -25°C. Plus précisément, la zone dite à basse température est par exemple comprise entre 0 et -25°C tandis que la zone à plus haute température est comprise entre +10 et -5°C par exemple.

La cristallisation selon deux niveaux de température peut être réalisée selon au moins deux variantes :

Conformément à la première variante, on peut cristalliser les xylènes provenant de l'étape de distillation dans une première zone de cristallisation à basse température, on sépare des premiers cristaux de paraxylène d'une première liqueur mère que l'on envoie dans la zone d'adsorption, on fond les premiers cristaux, on les recristallise dans une seconde zone de cristallisation à plus haute température, on sépare des seconds cristaux de paraxylène d'une seconde liqueur mère, on les lave avec un solvant de lavage adéquat, on les fond, on récupère du paraxylène de très haute pureté et on recycle une partie au moins de la seconde liqueur mère et éventuellement une partie au moins de la liqueur de lavage dans la première zone de cristallisation et/ou dans la deuxième zone de cristallisation et/ou dans la zone d'adsorption.

Conformément à une deuxième variante on peut cristalliser les xylènes provenant de l'étape de distillation dans une première zone de cristallisation à haute température, on sépare des premiers cristaux de paraxylène d'une première liqueur mère, on recristallise la première liqueur mère dans une deuxième zone de cristallisation à plus basse température, on sépare des seconds cristaux de paraxylène d'une seconde liqueur mère que l'on envoie dans la zone d'adsorption, on lave les premiers et les seconds cristaux avec un solvant de lavage adéquat, on les fond et on récupère du paraxylène de très haute pureté et on recycle une partie au moins de la liqueur de lavage dans la première ou dans la seconde zone de cristallisation et/ou dans la zone d'adsorption.

Selon un autre mode de mise en oeuvre du procédé, le paraxylène après avoir été cristallisé et séparé de sa liqueur mère peut être remis en suspension dans une zone de fusion partielle comme décrit dans la demande de brevet FR 95/00746 incorporée comme référence.

De manière plus détaillée, dans une première variante, on peut cristalliser les xylènes provenant de l'étape de distillation selon l'étape (c) de cristallisation dans la zone de cristallisation qui délivre une suspension de cristaux de paraxylène dans la liqueur mère, on sépare lesdits cristaux de la liqueur mère, on les fond partiellement dans une zone de fusion partielle, on récupère une seconde suspension de cristaux, on sépare et on lave ladite seconde suspension dans une zone de séparation et de lavage avec un solvant de lavage qui est le toluène ou du paraxylène fondu de très haute pureté, on récupère du paraxylène de très haute pureté que l'on fond éventuellement et une liqueur de lavage que l'on recycle au moins en partie dans la zone de cristallisation et/ou dans la zone d'adsorption après l'avoir éventuellement distillée si le solvant de lavage est du toluène.

Dans une deuxième variante, on peut cristalliser les xylènes provenant de l'étape de distillation, dans une première zone de cristallisation à haute température, on sépare des premiers cristaux de paraxylène d'une première liqueur mère, on recristallise la première liqueur mère dans une deuxième zone de cristallisation à plus basse température, on sépare des seconds cristaux de paraxylène d'une seconde liqueur mère que l'on envoie dans la zone d'adsorption, on lave les premiers et les seconds cristaux avec un solvant de lavage qui est le toluène ou du paraxylène fondu de très haute pureté, on refond partiellement les premiers et seconds cristaux dans au moins une zone de fusion partielle, on récupère une suspension de cristaux, on sépare et on lave ladite suspension dans une zone de séparation et de lavage avec un solvant de lavage qui est le toluène ou du paraxylène fondu de très haute pureté, on récupère du paraxylène de très haute pureté que l'on fond éventuellement et une liqueur de lavage et on recycle au moins en partie ladite liqueur de lavage dans la première et/ou la deuxième zone de cristallisation, après l'avoir éventuellement distillée si le solvant de lavage est du toluène, et/ou dans la zone d'adsorption.

La partie restante de la liqueur de lavage non recyclée vers la cristallisation ou l'adsorption peut être introduite dans la zone de fusion partielle où les cristaux obtenus, à plus petite taille, vont être remis en suspension.

L'adsorption de la liqueur mère sur un adsorbant sélectif peut être réalisée en lit mobile simulé à au moins 3 zones. Ce peut être un lit mobile à contre-courant simulé (US 2 985 589) ou à co-courant simulé (US 4 498 991 et US 4 402 832), ces brevets étant incorporés comme références.

Selon une première variante, l'adsorbant de la zone d'adsorption peut être un adsorbant sélectif pour le paraxylène. Ce peut être de préférence par exemple une zéolithe Y échangée au baryum et au potassium au moins en partie, comme décrit dans le brevet US 3 894 109 incorporé comme référence et le brevet US 3 558 732 citant le toluène comme désorbant. Selon cette variante, l'extrait récupéré contenant la fraction enrichie en paraxylène et le désorbant sont recyclés à l'étape de distillation (C1 ou C2) en amont de l'étape de cristallisation, tandis que le raffinat enrichi en ortho- et métaxylène et éthylbenzène et le désorbant sont traités dans une unité de distillation (C4), le désorbant étant ensuite recyclé dans l'unité d'adsorption.

Selon une deuxième variante, l'adsorbant peut être sélectif pour la fraction appauvrie en paraxylène et contenant le méta- et l'orthoxylène et l'éthylbenzène conformément aux brevets US 4 940 830 et US 5 382 742 incorporés comme références. Selon cette variante, le raffinat contenant le paraxylène et le désorbant sont recyclés dans l'unité de distillation en amont de la cristallisation tandis que l'extrait contenant les autres xylènes et l'éthylbenzène et le désorbant sont traités dans l'unité de distillation (C4), le désorbant étant ensuite recyclé dans l'unité d'adsorption.

Le schéma selon l'art antérieur comprenant une dismutation parasélective du toluène et une cristallisation à 1 ou 2 étages implique une cristallisation à très basse température si l'on désire atteindre un rendement très élevé en paraxylène, ce qui est extrêmement coûteux en énergie. Malgré tout, la perte en paraxylène peut atteindre jusqu'à 3 % en poids de paraxylène suivant la température de cristallisation adoptée. En interposant selon l'invention une adsorption traitant la liqueur mère recueillie de la cristallisation, on évite cette cristallisation à très basse température et on limite les pertes de paraxylène à moins de 0,1 % poids.

D'autre part, en utilisant le toluène comme désorbant on peut le régénérer dans une des colonnes traitant l'effluent de dismutation sans qu'il soit nécessaire d'ajouter une colonne spécifique de distillation pour séparer le paraxylène du désorbant.

L'invention sera mieux comprise au vu des figures suivantes qui illustrent de manière schématique quelques exemples de mise en oeuvre du procédé conduisant à la préparation de paraxylène de très haute pureté, parmi lesquelles :
- la figure 1 montre l'enchaînement des étapes du procédé : dismutation, fractionnement, cristallisation et purification du paraxylène et adsorption du paraxylène de la liqueur mère,
- la figure 2 illustre l'enchaînement avec une purification du paraxylène par un lavage au paraxylène, précédée d'une remise en suspension des cristaux,
- la figure 3 représente un mode de réalisation particulier du fractionnement par distillation,
- et la figure 4 reprend un fractionnement par distillation identique à celui de la figure 3 mais avec une séparation préalable des hydrocarbures C9+ en amont de la cristallisation.

Du toluène amené par une ligne la résultant d'une ligne 1 et d'une ligne de recyclage 7a est introduit dans un réacteur 2 de dismutation parasélective du toluène pour produire du benzène et des xylènes et plus particulièrement du paraxylène.

Un effluent est récupéré, refroidi et traité à la terre après séparation de l'hydrogène qui est recyclé et élimination des hydrocarbures légers (non représenté sur la figure).

L'effluent du réacteur contenant après ce traitement environ 15,5 % poids de xylènes et plus lourds, dont 80 % de paraxylène, 16,5 % de benzène et 68 % de toluène n'ayant pas réagi est acheminé par une ligne 3 et introduit dans une colonne de distillation (C1), pour produire un distillat constitué de benzène par une ligne 5 et un résidu contenant du toluène, des xylènes, de l'éthylbenzène et des hydrocarbures à au moins 9 atomes de carbone (C9+) par une ligne 6. Ce résidu alimente une colonne de distillation (C2) de laquelle est soutiré en partie du toluène en tant que distillat par une ligne 7, dont une partie est recyclée au réacteur 2 par la ligne 7a, tandis que l'autre partie du toluène est récupérée par une ligne 7b pour des lavages ultérieurs des cristaux de paraxylène, ou en tant que désorbant. En tant que résidu de la colonne (C2), on récupère des xylènes et des hydrocarbures (C9+) par une ligne 8.

Cette ligne 8 alimente une colonne (C3) de distillation adaptée à délivrer un résidu C9+ par une ligne 21 et un distillat par une ligne 9, contenant essentiellement l'ortho- et le métaxylène, le paraxylène et l'éthylbenzène.

Ce distillat est introduit par la ligne 9 dans une zone de cristallisation comprenant au moins un cristalliseur opérant à une température de +10 à -25°C par exemple. Des cristaux de paraxylène en suspension dans une liqueur mère appauvrie en paraxylène et contenant du métaxylène et de l'éthylbenzène sont recueillis par une ligne 11 et séparés dans une centrifugeuse 12. Les cristaux sont lavés dans la centrifugeuse en présence d'un solvant de lavage qui est du toluène amené par une ligne 24 connectée à la ligne 7b de sortie de la colonne de distillation (C2). Une liqueur de lavage contenant des impuretés et du toluène est recyclée par une ligne 12a vers l'unité de distillation (C2) permettant de séparer le toluène. Les cristaux purs de paraxylène recueillis de la centrifugeuse qui contiennent encore du toluène sont fondus dans un fondoir (non représenté sur la figure) puis introduits par une ligne 13 dans une colonne de distillation (C5). Un distillat de toluène est recyclé par une ligne 22 et la ligne 24 dans le dispositif de lavage de la centrifugeuse tandis que le paraxylène de très haute pureté est soutiré comme résidu de la colonne (C5), par une ligne 25.

La liqueur mère recueillie de la centrifugeuse 12 appauvrie en paraxylène et contenant en outre de l'éthylbenzène, du métaxylène et de l'orthoxylène est introduite par une ligne 14 dans une zone d'adsorption 15, constituée par douze lits d'adsorbant et fonctionnant suivant le principe du contre-courant simulé, comme décrit dans le brevet US 5 284 992. L'adsorbant est une zéolithe Y échangée par du baryum et du potassium. Du toluène provenant de la colonne de distillation (C2) par les lignes 7 et 7b, de la colonne de purification du toluène (C5) par une ligne 22a et/ou de la colonne de distillation (C4) définie ci-après par une ligne 18, est introduit comme éluant ou solvant de désorption, par une ligne 7c dans la zone d'adsorption. Un raffinat contenant du toluène, appauvri en paraxylène et enrichi en méta- et/ou orthoxylène et en éthylbenzène est recueilli par une ligne 16. Un extrait contenant du toluène et enrichi en paraxylène est désorbé et recyclé par une ligne 17 vers la colonne de distillation (C2) du toluène.

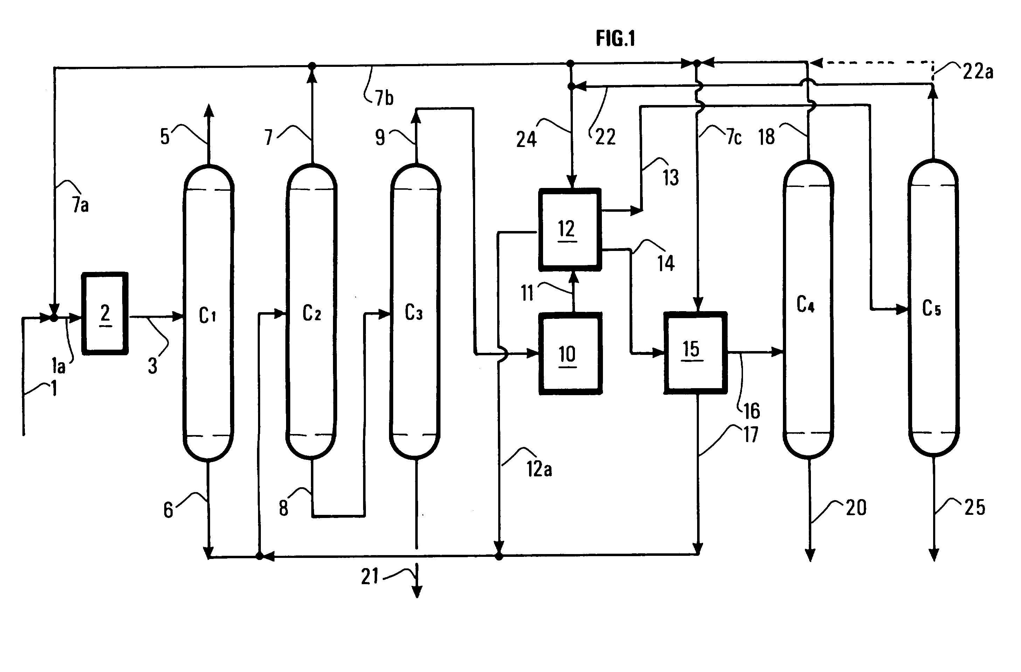

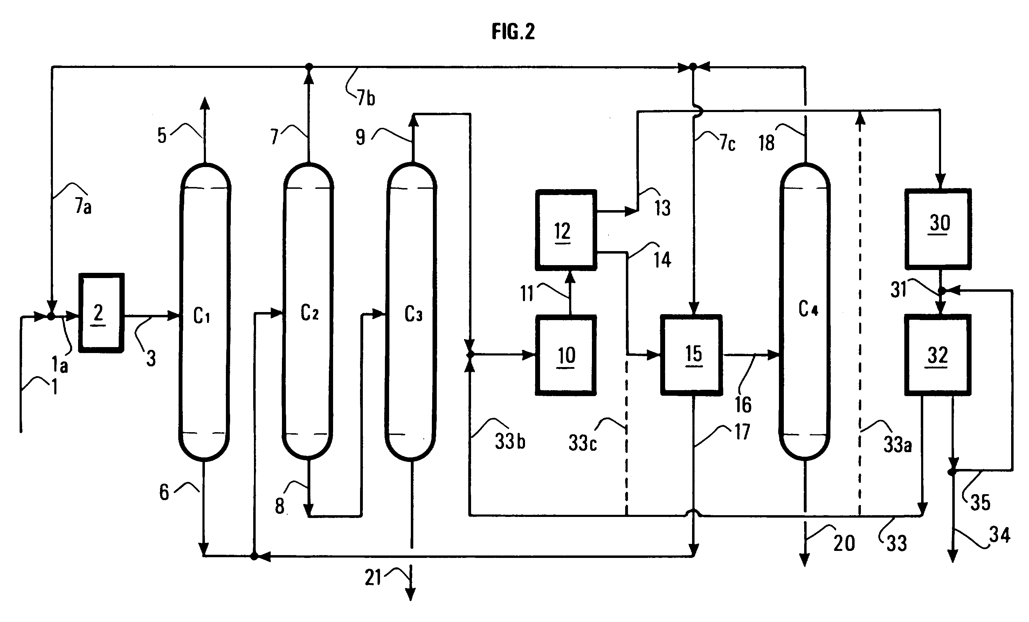

Selon la figure 2 qui reprend les mêmes références que celles de la figure 1 pour les mêmes organes, la suspension de cristaux de paraxylène dans la liqueur mère provenant de l'unité de cristallisation 10 est introduite par la ligne 11 dans la centrifugeuse 12. La liqueur mère recueillie est traitée conformément à la figure 1 tandis que les cristaux de paraxylène sont remis en suspension dans une zone de fusion partielle 30 où ils sont introduits par la ligne 13.

La suspension nouvelle recueillie par la ligne 31 est lavée dans une colonne de lavage à contre-courant 32, type NIRO par exemple comme décrit dans les brevets US 4 475 355, US 4 481 169, CH 515 730 et FR 95/00 746, par une partie du paraxylène fondu de très haute pureté récupéré de la colonne par une ligne 35. Une partie au moins d'une liqueur de lavage contenant du paraxylène est récupérée par la ligne 33 et recyclée dans la zone de cristallisation 10 par la ligne 33b et/ou dans la zone d'adsorption 15 par une ligne 33c tandis que la partie restante peut être introduite par une ligne 33a dans la ligne 13 et facilite ainsi la remise en suspension de cristaux et leur transfert dans la zone de fusion partielle 30. De préférence, la liqueur de lavage est recyclée dans la zone de cristallisation 10 et la zone de fusion partielle 30 en raison d'une teneur élevée en paraxylène.

Enfin, on récupère par une ligne 34 du paraxylène de très haute pureté.

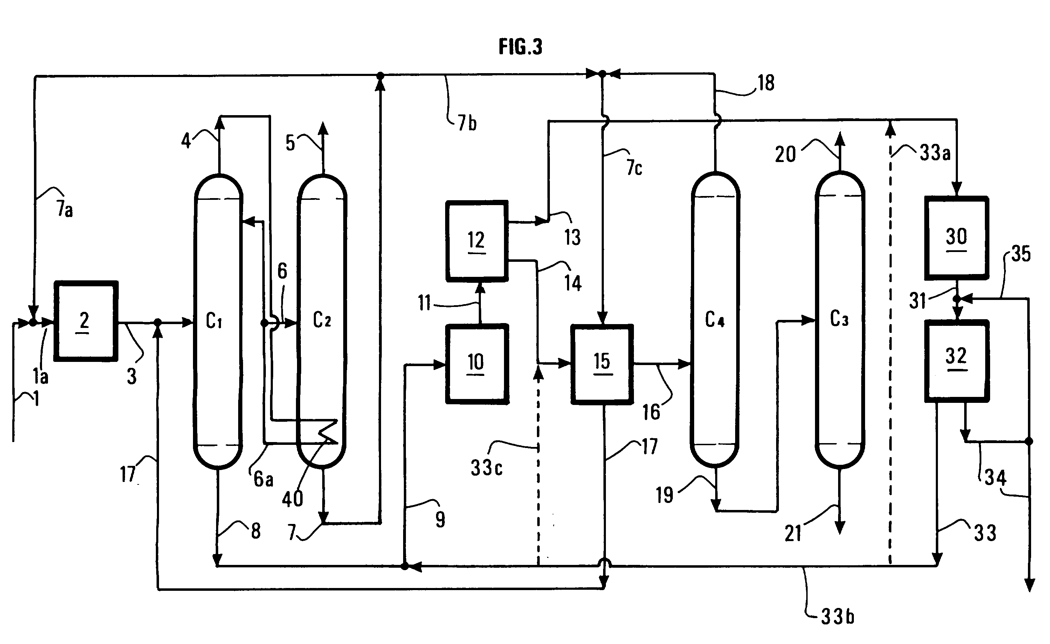

La figure 3 montre une variante de l'étape de distillation, les étapes de cristallisation du paraxylène et de purification des cristaux étant les mêmes que celles de la figure 1 ou celles de la figure 2.

Les références des divers organes communs aux dispositifs des figures 1 et 2 sont les mêmes.

Un mélange 317 de l'effluent 3 du réacteur de dismutation parasélective du toluène et du contenu d'une ligne 17 définie ci-après est distillé dans une colonne de distillation (C1) adaptée à délivrer un résidu contenant des xylènes et des hydrocarbures à au moins 9 atomes de carbone qui est envoyé dans la zone de cristallisation par la ligne 8. La colonne (C1) délivre aussi un distillat vapeur contenant du benzène et du toluène qui est condensé en cédant sa chaleur de condensation au rebouilleur d'une colonne C2 de distillation. Après avoir été condensé, ce distillat est recyclé en partie par une ligne 6a comme reflux en tête de colonne (C1), tandis que la partie restante alimente par une ligne 6 la colonne (C2). On récupère comme distillat du benzène par une ligne 5 et comme résidu du toluène par les lignes 7, 7a qui sont raccordées au réacteur de dismutation 2 et par les lignes 7 et 7b aux colonnes d'adsorption 15 et/ou au dispositif de lavage des cristaux une fois séparés (ligne 24, fig. 1).

Les cristaux de paraxylène séparés sont envoyés comme dans la figure 2 dans la zone de fusion partielle et purifiés comme il a été déjà indiqué selon cette figure 2.

La liqueur mère résultant de l'étape de centrifugation est envoyée dans les colonnes d'adsorption 15. L'extrait en résultant est recyclé dans la colonne de distillation C1 par la ligne 17 tandis que le raffinat appauvri en paraxylène mais contenant les hydrocarbures C9+ est distillé dans la colonne de distillation (C4) pour en éliminer sensiblement tout le toluène (ligne 18). Le résidu contenant aussi les hydrocarbures à au moins 9 atomes de carbone est envoyé par une ligne 19 dans une colonne de distillation (C3) de dimension réduite de laquelle sont soutirés en tant que résidu ces hydrocarbures par une ligne 21 et en tant que distillat l'ortho- et le méta-xylène et l'éthylbenzène.

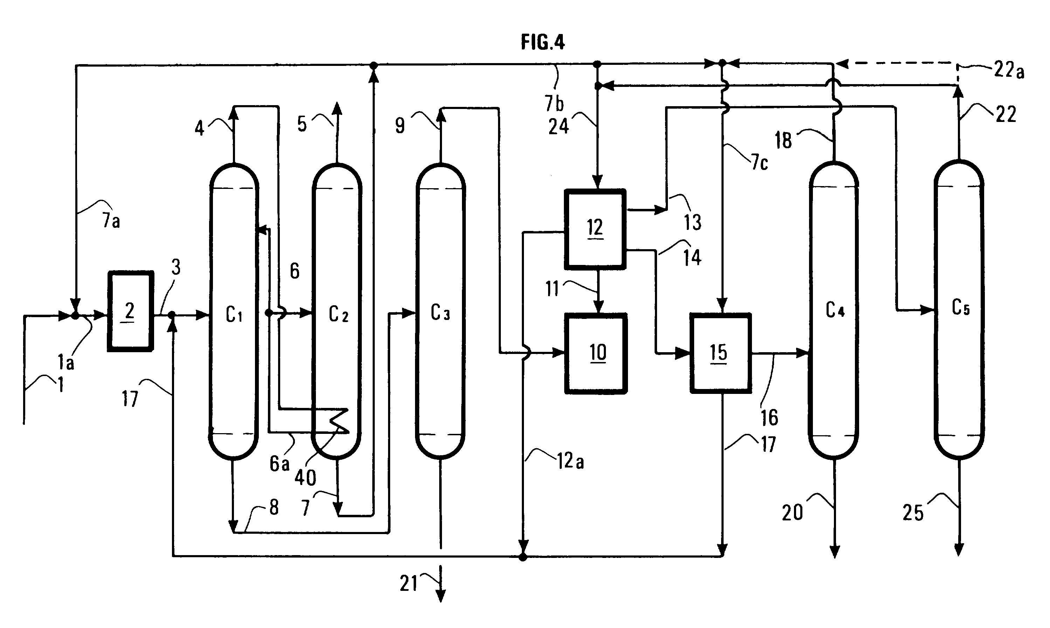

Selon la figure 4, au lieu d'enlever les hydrocarbures à au moins 9 atomes de carbone après l'étape de distillation du raffinat, comme dans la figure 3, on les retire avant l'étape de cristallisation. A cette fin, on alimente la colonne (C3) de distillation de dimension plus grande que celle selon la figure 3 par le résidu de la colonne (C1) contenant les xylènes et lesdits hydrocarbures à au moins 9 atomes de carbone. On récupère par la ligne 21 un résidu ne contenant que ces hydrocarbures et par une ligne 9 les xylènes que l'on introduit dans la zone de cristallisation 10. Les cristaux de paraxylène, une fois séparés de la liqueur mère et lavés sont purifiés comme il a été indiqué sur la figure 1 dans la colonne (C5), le toluène étant recyclé par la ligne 22 dans la centrifugeuse-laveuse. La liqueur de lavage en résultant est recyclée par les lignes 12a et 17 dans la colonne C1 de distillation.

L'exemple ci-après illustre l'invention.

Le tableau I présente un bilan matière du procédé décrit selon la figure 3, les différentes lignes reprenant les références de cette figure.

Les conditions opératoires des diverses étapes sont résumées ci-dessous :
Réacteur de dismutation parasélective
(MSTDP de Mobil)
Catalyseur ZSM5 activée
H₂/hydrocarbures (molaire) : 1,5
- Température :: 450°C
- Pression :: 25 bar
- PPH :: 3,5
Colonnes de distillation :
- (C1) :: 70 plateaux ; reflux 1,5
- (C2) :: 70 plateaux ; reflux 2,5
- (C3) :: 70 plateaux ; reflux 1,5
- (C4) :: 70 plateaux ; reflux 1,5
Cristalliseur
- Température :: -20°C à pression atmosphérique
- Fusion partielle :: 7°C à pression atmosphérique

Adsorption : contre-courant simulé
- Tamis :: Zéolithe Y échangée Ba et K
- Toluène/charge :: 1,2
- Température :: 150°C
- Pression :: 10-15 bar
- Lavage des cristaux :: paraxylène fondu

Dans ces conditions, on récupère la quasi-totalité du paraxylène produit dans le réacteur de dismutation, à une pureté de 99,9 %.

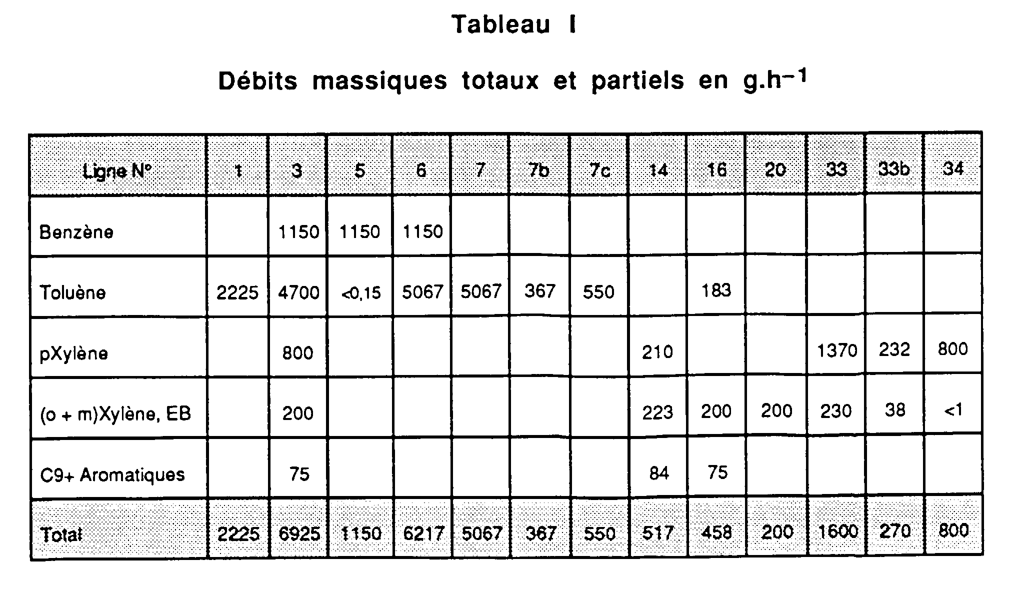

## Revendications

1. Procédé de préparation de paraxylène à partir d'une charge de toluène comprenant :
a) une étape de dismutation parasélective du toluène en présence d'hydrogène et d'un catalyseur dans une zone de dismutation délivrant un effluent contenant du benzène, du toluène, des xylènes enrichis en paraxylène, de l'éthylbenzène et des hydrocarbures aromatiques ayant au moins 9 atomes de carbone ;
b) une étape de distillation de l'effluent dans au moins deux colonnes de distillation délivrant séparément du benzène, du toluène et des xylènes ;
c) une étape de cristallisation des xylènes à haute température, de préférence comprise entre +10 et -30°C, dans au moins une zone de cristallisation délivrant du paraxylène de très haute pureté et une liqueur mère appauvrie en paraxylène, le procédé étant caractérisé en ce que :
d) on met en contact dans au moins une zone d'adsorption en lit mobile simulé ladite liqueur mère avec un lit d'adsorbant zéolithique en présence d'un désorbant qui est le toluène, dans des conditions telles qu'on obtient une première fraction appauvrie en paraxylène et contenant du toluène et une deuxième fraction enrichie en paraxylène contenant du toluène ;
e) on recycle au moins une partie de ladite deuxième fraction dans la zone de cristallisation après en avoir distillé sensiblement tout le toluène.

2. Procédé selon la revendication 1 dans lequel l'étape (b) de distillation comprend la distillation de l'effluent de l'étape (a) dans une première colonne de distillation (C1) qui délivre un distillat contenant du benzène et un premier résidu, la distillation du premier résidu dans une deuxième colonne de distillation (C2) qui délivre un distillat contenant du toluène et un second résidu, et la distillation du second résidu dans une troisième colonne de distillation (C3) qui délivre un distillat contenant les xylènes et un troisième résidu contenant les hydrocarbures aromatiques à au moins 9 atomes de carbone et dans lequel une partie au moins de ladite deuxième fraction est distillée dans la deuxième colonne de distillation (C2).

3. Procédé selon la revendication 2 dans lequel le troisième résidu est distillé en vue d'obtenir un distillat contenant de l'orthoxylène et un résidu contenant les hydrocarbures à au moins 9 atomes de carbone.

4. Procédé selon la revendication 1, dans lequel l'étape (b) de distillation comprend la distillation de l'effluent de l'étape (a) dans une première colonne de distillation (C1) qui délivre d'une part un distillat (BT) contenant du benzène et du toluène et d'autre part un résidu contenant les xylènes et les hydrocarbures à au moins 9 atomes de carbone, on soumet ledit résidu à l'étape (c) de cristallisation, on distille une partie au moins de ladite deuxième fraction enrichie en paraxylène dans la colonne de distillation (C1), on distille le distillat (BT) contenant le benzène et le toluène dans une colonne de distillation (C2) de façon à récupérer en tant que distillat du benzène et comme résidu du toluène et dans lequel une partie au moins du distillat (BT) de la colonne (C1) est utilisé pour apporter la chaleur nécessaire au rebouilleur de la colonne (C2).

5. Procédé selon l'une des revendications 1 à 4 dans lequel une partie au moins du toluène provenant de la colonne de distillation (C2) est recyclée à la zone d'adsorption, la partie restante étant recyclée dans la zone de dismutation.

6. Procédé selon la revendication 4 dans lequel le résidu de la colonne (C1) est distillé dans une colonne de distillation (C3) et on récupère un distillat contenant les xylènes que l'on soumet à l'étape (c) de cristallisation et un résidu contenant les hydrocarbures à au moins 9 atomes de carbone.

7. Procédé selon l'une des revendications 1 à 6 dans lequel la première fraction appauvrie en paraxylène contenant le toluène est distillée dans une colonne de distillation (C4) qui délivre comme distillat du toluène que l'on recycle au moins en partie dans la zone d'adsorption et un résidu contenant la première fraction sensiblement dépourvue de toluène.

8. Procédé selon l'une des revendications 1, 4 à 5 dans lequel on distille le résidu de la colonne de distillation (C4) dans une colonne de distillation (C3) de façon à obtenir un distillat contenant de l'orthoxylène, du métaxylène et de l'éthylbenzène et un résidu contenant les hydrocarbures à au moins 9 atomes de carbone.

9. Procédé selon l'une des revendications 1, 4 à 8 dans lequel une partie au moins de la deuxième fraction enrichie en paraxylène et contenant du toluène est recyclée dans la colonne (C1) de distillation.

10. Procédé selon l'une des revendications 2 à 3 dans lequel une partie au moins de la deuxième fraction enrichie en paraxylène et contenant du toluène est recyclée dans la colonne (C2).

11. Procédé selon l'une des revendications 1 à 10, dans lequel on cristallise les xylènes selon l'étape (c) dans une zone de cristallisation qui délivre une suspension de cristaux de paraxylène dans la liqueur mère, on sépare lesdits cristaux de la liqueur mère dans une zone de séparation, on lave les cristaux avec un solvant de lavage adéquat, on récupère la liqueur mère que l'on envoie dans la zone d'adsorption selon l'étape (d) et les cristaux de paraxylène de très haute pureté et on récupère une liqueur de lavage que l'on recycle dans la zone de distillation si le solvant de lavage contient du toluène ou dans la zone de cristallisation ou dans la zone d'adsorption si le solvant de lavage est du paraxylène fondu.

12. Procédé selon l'une des revendications 1 à 10 dans lequel on cristallise les xylènes selon l'étape (c) dans au moins deux zones de cristallisation dont l'une est à une température plus froide que l'autre, on récupère la liqueur mère provenant de la zone de cristallisation la plus froide et on l'envoie selon l'étape (d) dans la zone d'adsorption.

13. Procédé selon la revendication 12 dans lequel on cristallise les xylènes dans une première zone de cristallisation à basse température, on sépare des premiers cristaux de paraxylène d'une première liqueur mère que l'on envoie dans la zone d'adsorption, on fond les premiers cristaux, on les recristallise dans une seconde zone de cristallisation à plus haute température, on sépare des seconds cristaux de paraxylène d'une seconde liqueur mère, on les lave avec un solvant de lavage adéquat, on les fond, on récupère du paraxylène de très haute pureté et on recycle une partie au moins de la seconde liqueur mère et éventuellement une partie au moins de la liqueur de lavage dans la première zone de cristallisation et/ou dans la deuxième zone de cristallisation et/ou dans la zone d'adsorption.

14. Procédé selon la revendication 12, dans lequel on cristallise les xylènes dans une première zone de cristallisation à haute température, on sépare des premiers cristaux de paraxylène d'une première liqueur mère, on recristallise la première liqueur mère dans une deuxième zone de cristallisation à plus basse température, on sépare des seconds cristaux de paraxylène d'une seconde liqueur mère que l'on envoie dans la zone d'adsorption, on lave les premiers et les seconds cristaux avec un solvant de lavage adéquat, on les fond et on récupère du paraxylène de très haute pureté et on recycle une partie au moins de la liqueur de lavage dans la première ou dans la seconde zone de cristallisation et/ou dans la zone d'adsorption.

15. Procédé selon l'une des revendications 1 à 10 dans lequel on cristallise les xylènes selon l'étape (c) dans la zone de cristallisation qui délivre une suspension de cristaux de paraxylène dans la liqueur mère, on sépare lesdits cristaux de la liqueur mère, on les fond partiellement dans une zone de fusion partielle, on récupère une seconde suspension de cristaux, on lave et on sépare ladite seconde suspension dans une zone de séparation et de lavage avec un solvant de lavage qui est le toluène ou du paraxylène fondu de très haute pureté, on récupère du paraxylène de très haute pureté que l'on fond éventuellement et une liqueur de lavage que l'on recycle au moins en partie dans la zone de cristallisation et/ou dans la zone d'adsorption après l'avoir éventuellement distillée si le solvant de lavage est du toluène.

16. Procédé selon l'une des revendications 1 à 9 et 12 dans lequel on cristallise les xylènes dans une première zone de cristallisation à haute température, on sépare des premiers cristaux de paraxylène d'une première liqueur mère, on recristallise la première liqueur mère dans une deuxième zone de cristallisation à plus basse température, on sépare des seconds cristaux de paraxylène d'une seconde liqueur mère que l'on envoie dans la zone d'adsorption, on lave les premiers et les seconds cristaux avec un solvant de lavage qui est le toluène ou du paraxylène fondu de très haute pureté, on refond partiellement les premiers et seconds cristaux dans au moins une zone de fusion partielle, on récupère une suspension de cristaux, on sépare et on lave ladite suspension dans une zone de séparation et de lavage avec un solvant de lavage qui est le toluène ou du paraxylène fondu de très haute pureté, on récupère du paraxylène de très haute pureté que l'on fond éventuellement et une liqueur de lavage et on recycle au moins en partie ladite liqueur de lavage dans la première et/ou la deuxième zone de cristallisation, après l'avoir éventuellement distillée si le solvant de lavage est du toluène et/ou dans la zone d'adsorption.

17. Procédé selon la revendication 15 ou 16, dans lequel on recycle la partie restante de la liqueur de lavage dans la zone de fusion partielle.

18. Procédé selon l'une des revendications 11 à 17 dans lequel le solvant de lavage est le toluène, on distille le paraxylène fondu de très haute pureté contenant du toluène dans une colonne de distillation (C5) et on recycle le toluène en tant que solvant de lavage.

## Patentansprüche

1. Verfahren zur Herstellung von para-Xylol ausgehend von einer Toluolcharge umfassend:
a. einen paraselektiven Dismutationsschritt von Toluol in Gegenwart von Wasserstoff und eines Katalysators in einer Dismutationszone, einen Abstrom aus Benzol, Toluol, an para-Xylol reichen Xylolen, Ehtylbenzol und aromatischen Kohlenwasserstoffen, die mindestens 9 Kohlenstoffatome haben, liefernd;
b. einen Destillationsschritt des Abstroms in mindestens zwei Destillationssäulen, getrennt Benzol, Toluol und Xylole liefernd;
c. einen Kristallisationsschritt der Xylole bei hoher Temperatur, vorzugsweise zwischen +10 und -30°C in mindestens einer Kristallisationszone, para-Xylol sehr hoher Reinheit und eine an para-Xylol veramte Stammflüssigkeit liefernd, wobei das Verfahren **dadurch gekennzeichnet ist,** dass:
d. man zumindest in einer Zone der Adsorption im simulierten beweglichen Bett diese Stammflüssigkeit mit einem zeolithischen Adsorbensbett, in Gegenwart eines Desorptionsmittels, das Toluol ist, in Kontakt bringt, unter Bedingungen derart, dass man eine erste an para-Xylol verarmte und Toluol enthaltende Fraktion und eine zweite an para-Xylol angereicherte, Toluol enthaltende Fraktion erhält;
e. man gegebenenfalls zumindest einen Teil dieser zweiten Fraktion in die Kristallisationszone rezykliert, nachdem man davon im wesentlichen alles Toluol abdestilliert hat.

2. Verfahren nach Anspruch 1, in dem der Destillationsschritt (b) die Destillation des Abstroms des Schrittes (a) in einer ersten Destillationssäule (C1), die ein Benzol enthaltendes Destillat liefert, und einen ersten Rückstand, die Destillation des ersten Rückstands in einer zweiten Destillationssäule (C2), die ein Toluol enthaltendes Destillat liefert und einen zweiten Rückstand und eine dritte Destillationssäule (C3) umfasst, die ein die Xylole enthaltendes Destillat liefert und einen dritten, die aromatischen Kohlenwasserstoffe mit mindestens 9 Kohlenstoffatomen enthaltenden Rückstand und in dem ein Teil, zumindest der zweiten Fraktion in der zweite Destillationssäule (C2) destilliert wird.

3. Verfahren nach Anspruch 2, in dem der dritte Rückstand in Hinblick auf den Erhalt eines Destillats destilliert wird, welches ortho-Xylol und einen Kohlenwasserstoffe mit mindestens 9 Kohlenstoffatomen enthaltenden Rückstand umfasst.

4. Verfahren nach Anspruch 1, in dem der Destillationsschritt (b) die Destillation des Abstroms des Schrittes (a) in einer ersten Destillationssäule (C1) umfasst, die zum einen Teil ein Benzol und Toluol enthaltendes Destillat (BT) liefert und zum anderen Teil einen die Xylole und die Kohlenwasserstoffe mit mindestens 9 Kohlenstoffatomen enthaltenden Rückstand, man diesen Rückstand dem Kristallisationsschritt (c) unterzieht, man einen Teil, mindestens dieser zweiten, an para-Xylol angereicherten Fraktion in der Destillationssäule (C1) destilliert, man das Benzol und Toluol enthaltende Destillat (BT) in einer Destillationssäule (C2) derart destilliert, dass man als Destillat Benzol und als Rückstand Toluol gewinnt und wobei ein Teil, wenigstens des Destillats (BT) der Säule (C1) verwendet wird, um die nötige Hitze zum Verdampfer der Säule (C2) zu bringen.

5. Verfahren nach einem der Ansprüche 1 bis 4, in dem ein Teil, wenigstens des von der Destillationssäule (C2) kommenden Toluols zur Adsorptionszone rezykliert wird, wobei der verbleibende Teil in die Dismutationszone rezykliert wird.

6. Verfahren nach Anspruch 4, in dem der Rückstand der Säule (C1) in der Destillationssäule (C3) destilliert wird und man ein die Xylole umfassendes Destillat, welches man dem Kristallisationsschritt (c) unterzieht und einen die Kohlenwasserstoffe mit mindestens 9 Kohlenstoffatomen enthaltenden Rückstand aufnimmt.

7. Verfahren nach einem der Ansprüche 1 bis 6, in dem die erste an para-Xylol verarmte und Toluol enthaltende Fraktion in einer Destillationssäule (C4) destilliert wird, die als Destillat Toluol, das man zumindest teilweise in die Adsorptionszone rezykliert und einen Rückstand der im wesentlichen toluolfreien ersten Fraktion liefert.

8. Verfahren nach einem der Ansprüche 1, 4 bis 5, in dem man den Rückstand der Destillationssäule (C4) in einer Destillationssäule (C3) so destilliert, dass man ein ortho-Xylol, metha-Xylol und Ethylbenzol umfassendes Destillat erhält und einen einen die Kohlenwasserstoffe mit mindestens 9 Kohlenstoffatomen enthaltenden Rückstand.

9. Verfahren nach einem der Ansprüche 1, 4 bis 8, in dem wenigstens ein Teil der zweiten, an para-Xylol angereicherten und Toluol enthaltenden Fraktion in die Destillationssäule (Cl) rezykliert wird.

10. Verfahren nach einem der Ansprüche 2 bis 3, in dem ein Teil zumindest der zweiten, an para-Xylol angereicherten und Toluol enthaltenden Fraktion in die Säule (C2) rezykliert wird.

11. Verfahren nach einem der Ansprüche 1 bis 10, in dem man die Xylole gemäß dem Schritt (c) in einer Kristallisationszone kristallisiert, die eine Suspension von para-Xylol-Kristallen in der Stammflüssigkeit liefert, man diese Kristalle von der Stammflüssigkeit in einer Trennzone trennt, man die Kristalle mit einem geeigneten Waschlösungsmittel wäscht, man die Stammflüssigkeit aufnimmt, die man in die Destillationszone rezykliert, wenn das Waschlösungsmittel Toluol enthält oder in die Kristallisationszone oder in die Adsorptionszone, wenn das Waschlösungsmittel geschmolzenes para-Xylol ist.

12. Verfahren nach einem der Ansprüche 1 bis 10, in dem man die Xylole gemäß dem Schritt (c) in zumindest zwei Kristallisationszonen kristallisiert, von denen die eine bei einer kälteren Temperatur ist als die andere, man die von der kältesten Kristallisationszone kommende Stammflüssigkeit aufnimmt und man sie gemäß dem Schritt (d) in die Adsorptionszone leitet.

13. Verfahren nach Anspruch 12, in dem man die Xylole in einer ersten Kristallisationszone bei niedriger Temperatur kristallisiert, man erste para-Xylol-Kristalle von einer ersten Stammflüssigkeit trennt, die man in die Adsorptionszone leitet, man die ersten Kristalle schmilzt, man sie in einer zweiten Kristallisationszone bei höherer Temperatur regkristallisiert, man zweite para-Xylol-Kristalle von einer zweiten Stammflüssigkeit trennt, man sie mit einem geeigneten Waschlösungsmittel wäscht, man sie schmilzt, man para-Xylol sehr hoher Reinheit aufnimmt und einen Teil, zumindest der zweiten Stammflüssigkeit und gegebenenfalls einen Teil, zumindest der Waschflüssigkeit in die erste Kristallisationszone und/oder in die zweite Kristallisationszone und/oder in die Adsorptionszone rezykliert.

14. Verfahren nach Anspruch 12, in dem man die Xylole in einer ersten Kristallisationszone bei hoher Temperatur kristallisiert, man erste para-Xylol-Kristalle von einer ersten Stammflüssigkeit trennt, man die erste Stammflüssigkeit in einer zweiten Kristallisationszone bei niedrigerer Temperatur regkristallisiert, man zweite para-Xylol-Kristalle von einer zweiten Stammflüssigkeit trennt, die man in die Adsorptionszone leitet, man die ersten und die zweiten Kristalle mit einem geeigneten Waschlösungsmittel wäscht, man sie schmilzt und man para-Xylol sehr hoher Reinheit aufnimmt und einen Teil zumindest der Waschflüssigkeit in die zweite Kristallisationszone und/oder in die Adsorptionszone rezykliert.

15. Verfahren nach einem der Ansprüche 1 bis 10, in dem man die Xylole gemäß dem Schritt (c) in einer Kristallisationszone kristallisiert, die eine Suspension von para-Xylol-Kristallen in der Stammflüssigkeit liefert, man diese Kristalle von der Stammflüssigkeit trennt, man sie teilweise in einer Zone partiellen Schmelzens schmilzt, man eine zweite Kristallsuspension aufnimmt, man diese zweite Kristallsuspension in einer Trenn- und Waschzone mit einem Waschlösungsmittel wäscht und trennt, welches geschmolzenes Toluol oder para-Xylol sehr hoher Reinheit ist, man para-Xylol sehr hoher Reinheit aufnimmt, das man gegebenenfalls schmilzt und eine Waschflüssigkeit, die man zumindest teilweise in die Kristallisationszone und/oder in die Adsorptionszone rezykliert, nachdem man sie gegebenenfalls destilliert hat, wenn das Waschlösungsmittel Toluol ist.

16. Verfahren nach einem der Ansprüche 1 bis 9 und 12, in dem man die Xylole in einer ersten Kristallisationszone bei hoher Temperatur kristallisiert, man erste para-Xylol-Kristalle von einer ersten Stammflüssigkeit trennt, man die erste Stammflüssigkeit in einer zweiten Kristallisationszone bei niedrigerer Temperatur rekristallisiert, man zweite para-Xylol-Kristalle von einer zweiten Stammflüssigkeit trennt, die man in die Adsorptionszone leitet, man die ersten und die zweiten Kristalle mit einem geeigneten Waschlösungsmittel wäscht, welches Toluol oder geschmolzenes para-Xylol sehr hoher Reinheit ist, man die ersten und die zweiten Kristalle teilweise in wenigstens einer Zone partiellen Schmelzens schmilzt, man eine Kristallsuspension aufnimmt, man diese Suspension in einer Trenn- und Waschzone mit einem Waschlösungsmittel wäscht, welches Toluol oder geschmolzenes para-Xylol sehr hoher Reinheit ist, man para-Xylol sehr hoher Reinheit, das man gegebenenfalls schmilzt, und eine Waschflüssigkeit aufnimmt und man zumindest teilweise diese Waschflüssigkeit in die erste und/oder zweite Kristallisationszone rezykliert, nachdem man sie gegebenenfalls destilliert hat, wenn das Waschlösungsmittel Toluol ist und/oder in die Adsorptionszone.

17. Verfahren nach einem der Ansprüche 15 oder 16, in dem man den verbleibenden Teil der Waschflüssigkeit in die Zone partiellen Schmelzens rezykliert.

18. Verfahren nach einem der Ansprüche 11 bis 17, in dem das Waschlösungsmittel Toluol ist, man geschmolzenes para-Xylol sehr hoher Reinheit, welches Toluol enthält in einer Destillationssäule (C5) destilliert und man das Toluol als Waschlösungsmittel rezykliert.

## Claims

1. A process for the preparation of para-xylene from a toluene feed, comprising:
a) a paraselective toluene disproportionation step in the presence of hydrogen and a catalyst in a disproportionation zone to produce an effluent containing benzene, toluene, para-xylene enriched xylenes, ethylbenzene and aromatic hydrocarbons containing at least 9 carbon atoms;
b) an effluent distillation using at least two distillation columns to separately produce benzene, toluene and xylenes;
c) a high temperature xylene crystallization step, preferably in the range +10C to -30C, using at least one crystallization zone to produce very high purity para-xylene and a mother liquor which is depleted in para-xylene,
the process being characterized in that:
d) said mother liquor is brought into contact with a zeolitic adsorption bed in a simulated moving bed adsorption zone in the presence of a toluene desorbent under conditions such that a first fraction is obtained which is depleted in para-xylene and contains toluene and a second fraction is obtained which is enriched in para-xylene and contains toluene;
e) at least a portion of said second fraction is recycled to the crystallization zone after distilling substantially all of the toluene.

2. A process according to claim 1, in which distillation step (b) comprises distillation of the effluent from step (a) in a first distillation column (C1) to produce a distillate containing benzene and a first residue, distillation of the first residue in a second distillation column (C2) to produce a distillate containing toluene and a second residue, and distillation of the second residue in a third distillation column (C3) to produce a distillate containing xylenes and a third residue containing aromatic hydrocarbons containing at least 9 carbon atoms and in which at least a portion of said second fraction is distilled in the second distillation column (C2).

3. A process according to claim 2, in which the third residue is distilled to obtain a distillate containing ortho-xylene and a residue containing hydrocarbons containing at least 9 carbon atoms.

4. A process according to claim 1, in which distillation step (b) comprises distillation of the effluent from step (a) in a first distillation column (C1) to produce a distillate (BT) containing benzene and toluene and a residue containing xylenes and hydrocarbons containing at least 9 carbon atoms, said residue undergoes crystallization step (c), at least a portion of said second para-xylene enriched fraction is distilled in distillation column (C1), and distillate (BT) containing benzene and toluene is distilled in a distillation column (C2) to recover benzene as the distillate and toluene as the residue, and in which at least a portion of distillate (BT) from column (C1) is used to supply the necessary heat to the reboiler for column (C2).

5. A process according to any one of claims 1 to 4, in which at least a portion of the toluene from distillation column (C2) is recycled to the adsorption zone, the remaining portion being recycled to the disproportionation zone.

6. A process according to claim 4, in which the residue from column (C1) is distilled in a distillation column (C3) and a distillate containing xylenes is recovered which is subjected to crystallization step (c), also a residue containing hydrocarbons containing at least 9 carbon atoms is recovered.

7. A process according to any one of claims 1 to 6, in which the first fraction which is depleted in para-xylene, containing toluene, is distilled in a distillation column (C4) to produce toluene as a distillate, at least a portion of which is recycled to the adsorption zone, and a residue containing the first fraction which is substantially free of toluene.

8. A process according to any one of claims 1, 4 and 5, in which the residue from distillation column (C4) is distilled in a distillation column (C3) to obtain a distillate containing ortho-xylene, meta-xylene and ethylbenzene and a residue containing hydrocarbons containing at least 9 carbon atoms.

9. A process according to any one of claims 1, and 4 to 8, in which at least a portion of the second fraction which is enriched in para-xylene and contains toluene is recycled to distillation column (C1).

10. A process according to claim 2 or claim 3, in which at least a portion of the second fraction which is enriched in para-xylene and contains toluene is recycled to column (C2).

11. A process according to any one of claims 1 to 10, in which the xylenes are crystallized in accordance with step (c) in a crystallization zone to produce a suspension of para-xylene crystals in the mother liquor, said crystals are separated from the mother liquor in a separation zone, the crystals are washed with a suitable wash solvent, the mother liquor is recovered and sent to the adsorption zone in accordance with step (d) and very high purity para-xylene crystals are recovered, and the wash liquor is recovered and recycled to the distillation zone if the wash solvent contains toluene or to the crystallization zone or the adsorption zone if the wash solvent is molten para-xylene.

12. A process according to any one of claims 1 to 10, in which the xylenes are crystallized in accordance with step (c) in at least two crystallization zones, one of which is at a colder temperature than the other, the mother liquor from the coldest crystallization zone being recovered and sent to the adsorption zone in accordance with step (d).

13. A process according to claim 12, in which the xylenes are crystallized in a first low temperature crystallization zone, the first para-xylene crystals are separated from a first mother liquor which is sent to the adsorption zone, the first crystals are melted, they are recrystallised in a second higher temperature crystallization zone, second para-xylene crystals are separated from a second mother liquor, washed with a suitable wash solvent, melted, and very high purity para-xylene is recovered, and at least a portion of the second mother liquor and optionally at least a portion of the wash liquor is recycled to the first crystallization zone and/or to the second crystallization zone and/or to the adsorption zone.

14. A process according to claim 12, in which the xylenes are crystallized in a first high temperature crystallization zone, first para-xylene crystals are separated from a first mother liquor, the first mother liquor is recrystallised in a second lower temperature crystallization zone, second para-xylene crystals are separated from a second mother liquor which is sent to the adsorption zone, the first and second crystals are washed with a suitable wash solvent, melted, and very high purity para-xylene is recovered, and at least a portion of the wash liquor is recycled to the first crystallization zone or to the second crystallization zone and/or to the adsorption zone.

15. A process according to any one of claims 1 to 10, in which the xylenes are crystallized in accordance with crystallization step (c) in a crystallization zone to produce a suspension of para-xylene crystals in a mother liquor, said crystals are separated from the mother liquor, partially melted in a partial melting zone, a second suspension of crystals is recovered, said second suspension is separated and washed in a separation and washing zone using a wash solvent which is toluene or molten very high purity para-xylene, very high purity para-xylene is recovered which is optionally melted, and a wash liquor is recovered which is at least partially recycled to the crystallization zone and/or to the adsorption zone after optional distillation if the wash solvent is toluene.

16. A process according to any one of claims 1 to 9 and 12, in which the xylenes are crystallized in a first high temperature crystallization zone, first crystals of para-xylene are separated from a first mother liquor, the first mother liquor is recrystallised in a second lower temperature crystallization zone, second para-xylene crystals are separated from a second mother liquor which is sent to the adsorption zone, the first and second crystals are washed with a wash solvent which is toluene or molten very high purity para-xylene, the first and second crystals are partially melted in at least one partial melting zone, a suspension of crystals is recovered, said suspension is separated and washed in a separation and washing zone with a wash solvent which is toluene or molten very high purity para-xylene, very high purity para-xylene is recovered and optionally melted and a wash liquor is recovered, and at least a portion of the wash liquor is recycled to the first and/or second crystallization zone after optional distillation if the wash solvent is toluene, and/or to the adsorption zone.

17. A process according to claim 15 or claim 16, in which the remaining portion of the wash liquor is recycled to the partial melting zone.

18. A process according to any one of claims 11 to 17, in which the wash solvent is toluene, the very high purity para-xylene containing toluene is distilled in a distillation column (C5) and the toluene is recycled as the wash solvent.
